# EUROPEAN PATENT SPECIFICATION

(11) **EP 2 242 383 B1**
(45) Date of publication and mention of the grant of the patent: **08.10.2014**
(21) Application number: 08864349.9
(22) Date of filing: 19.12.2008
(51) Int. Cl.: A23L 1/308, A61K 31/715

(54) **INDUCED VISCOSITY NUTRITIONAL EMULSIONS COMPRISING A CARBOHYDRATE-SURFACTANT COMPLEX**
EMULSIONEN MIT INDUKTIVER VISCOSITÄT ENTHALTEND EINEN KOMPLEX AUS EINEM GLUZID UND EINEM TENSID
ÉMULSIONS NUTRITIONNELLES À VISCOSITÉ INDUITE COMPRENANT UN COMPLEXE DE GLUCIDE-AGENT TENSIOACTIF

(30) Priority: 21.12.2007 US 962201
(43) Date of publication of application: 27.10.2010
(73) Proprietor: ABBOTT LABORATORIES, Abbott Park, IL 60064-3500 (US)
(72) Inventor: LAI, Chron-si, Blacklick OH 43004 (US); WALTON, Joseph, Westerville OH 43081 (US); WEN-LIU, James, Dublin OH 43016 (US); DRAPER, Kati, Terre Haute, IN 47802 (US)
(74) Representative: Modiano, Micaela Nadia
(86) International application number: PCT/US2008/087595
(87) International publication number: WO 2009/082680

(56) References cited:
- US-A- 4 491 483
- US-A- 5 462 742
- US-A1- 2002 152 931
- US-A1- 2003 125 301
- US-A1- 2003 134 027
- US-A1- 2005 233 045

## Description

### TECHNICAL FIELD

The present invention relates to induced viscosity nutritional emulsions comprising a carbohydrate-surfactant complex (V-complex) for improved satiety benefits.

### BACKGROUND OF THE INVENTION

There are many different types of nutritional emulsions commercially available or are otherwise disclosed in the literature. These are typically oil-in-water emulsions comprising a balance of fat, protein, carbohydrate, vitamins, and minerals. Some examples include Glucerna® and Ensure® brands of packaged nutritional liquids, available from Abbott Laboratories, Columbus, Ohio.

Recently, a new type of nutritional liquid has been developed that contains, as part of a carbohydrate component, an induced viscosity fiber system. These liquids have a packaged viscosity typical of a nutritional emulsion, but because of the fiber system, result in a higher induced viscosity following consumption. The increased viscosity in the stomach helps reduce gastric emptying and the subsequent blood glucose response. The viscosity increase within the stomach also provides a sense of fullness and enhanced satiety. These induced viscosity beverages are especially useful in diabetics and in people interested in maintaining or losing weight.

For example, U.S. Patent Publication 2002/0193344 (Wolf et al.) discloses an induced viscosity beverage, wherein the beverage contains an induced viscosity fiber system having a soluble, anionic fiber in combination with a water insoluble, acid-soluble cation. The viscosity of the beverage increases following consumption when exposed to the low pH of the stomach.

In yet another example, U.S. Patent 7,067,498 (Wolf et al.) discloses an induced viscosity beverage, wherein the beverage comprises a partially hydrolyzed starch in combination with a neutral soluble fiber. The viscosity of the beverage increases following consumption when exposed to acid and amylase in the stomach.

It is known that induced viscosity emulsions may be used to control postprandial appetite. Since postprandial hunger increases when at least about 60% of an ingested meal has emptied from the stomach, induced viscosity emulsions may be used to reduce hunger by increasing gastric viscosity, which then reduces gastric emptying rates, which then reduces hunger. Increased gastric viscosity resists the propulsive contractions of the stomach due to their greater inertia allowing them to empty more slowly than nonviscous contents.

It is also known that low calorie foods have little effect on controlling postprandial hunger. It has been found that these low calorie foods, even when formulated as induced viscosity emulsions as described above, have little effect on controlling posprandial hunger. And because induced viscosity emulsions are often described for use in reducing or maintaining body weight, it would be desirable to formulate such emulsions as low calorie foods, provided that such foods could also be formulated to control appetite.

There is therefore a need to formulate induced viscosity emulsions that are even more effective in controlling appetitive, especially when formulated as a low calorie or low fat formulation.

### SUMMARY OF THE INVENTION

One embodiment of the present invention includes nutritional emulsions comprising fat, protein, and carbohydrate, including: (A) an induced viscosity fiber system, and (B) a V-complex located within an aqueous phase of the emulsion and comprising a food grade surfactant complexed with a polydextrose having an average degree of polymerization of at least about 10, wherein the nutritional emulsions have a first viscosity at 20° C of less than about 100 cps, a second viscosity at a temperature of from 0° C to 8° C that is at least about 50 cps higher than said first viscosity, and an induced viscosity of at least about 300 cps.

The V-complex component of the induced viscosity beverage helps provide consumers with increased satiety even when the beverage is formulated as a low fat or low calorie emulsion. It is believed that the acylglycerol component of the V-complex acts as a CCK/GLP-1 stimulating agent, the effect of which (on satiety) is much greater than comparable formulations containing the same acylglycerol but not harbored within or complexed to a V-complex.

The embodiments of the present invention may also provide nutritional emulsions that have a lower viscosity during processing and storage, but a surprisingly higher viscosity when chilled. The lower processing viscosity allows for the use of less severe processing temperatures and/or shear. The higher chilled viscosity provides a surprisingly thick and creamy texture or mouthfeel, similar to a formulation with a higher fat content.

### DETAILED DESCRIPTION OF THE INVENTION

The various embodiments of the present invention may include nutritional emulsions, and methods for making those emulsions, all of which may comprise an induced viscosity fiber system, and selected V-complexes as defined herein and/or an aqueous phase comprising the V-complexes or selected combinations of a polydextrose and a food grade surfactant. These and other essential or optional elements of the various embodiments are described in detail hereinafter.

The term "nutritional emulsion" as used herein, unless otherwise specified, means a room temperature emulsion comprising fat, protein, and carbohydrates, that is suitable for use as a sole, primary, or supplemental source of oral nutrition in a human. Such nutritional emulsions include classic emulsions (e.g., complex, water-in-oil, oil-in-water, etc.), suspensions (e.g., suspended solids), and combinations thereof. The nutritional emulsions are most typically oil-in-water emulsions having a continuous aqueous phase and a discontinuous oil phase.

Viscosity values as used herein, unless otherwise specified, are obtained using a Brookfield Viscometer (Model DV-II+) with a 62 spindle at room temperature (20° C), or at the temperature so designated. The viscosity is measured by operating the viscometer at a spindle speed that is the highest speed possible to obtain a reading that is on scale. The measured viscosity values represent the ratio of shear stress to shear rate, expressed as dynes-second/cm2, or poise, or more typically as centipoise (cps) or one hundredth of a poise.

All percentages, parts and ratios as used herein are by weight of the total composition, unless otherwise specified. All such weights as they pertain to listed ingredients are based on the active level and, therefore, do not include solvents or by-products that may be included in commercially available materials, unless otherwise specified.

Any reference to singular characteristics or limitations of the present invention shall include the corresponding plural characteristic or limitation, and vice versa, unless otherwise specified.

Any combination of method or process steps as used herein can be performed in any order, unless otherwise specified.

The embodiments of the compositions and methods of the present invention may be substantially free of any specific ingredient described herein, provided that the remaining composition comprises all of the essential limitations as defined herein. In this context, the term "substantially free" means that the compositions may comprise less than a functional amount of the identified ingredient disclosed herein, typically less than about 1%, including less than about 0.5%, also including less than about 0.1%, and also including zero percent, by weight of the identified ingredient.

The embodiments of the compositions and methods of the present invention may comprise, consist of, or consist essentially of the essential elements of the invention described herein, as well as any additional or optional ingredients or components described herein or otherwise useful in a nutritional application.

The term "induced viscosity nutritional emulsion" as used herein, unless otherwise specified, refers to a nutritional emulsion comprising an induced viscosity fiber system as defined herein.

The term "induced viscosity fiber system" as used herein, unless otherwise specified, means any fiber-containing material or composition, that when added to a nutritional emulsion allows for a drinkable emulsion viscosity at 20° C of less than about 300 cps and an increased (induced) emulsion viscosity following consumption.

The term "food grade surfactant" as used herein, unless otherwise specified, means the surfactant component of the carbohydrate-surfactant complexes (V-complexes) described herein.

### Viscosity Profile

The nutritional emulsion embodiments of the present invention have a viscosity profile as defined herein, wherein each has a defined viscosity at 20°C (room temperature or packaged viscosity), a defined viscosity increase when the product temperature rises from 20° C to within a range of about 0° to about 8° C (chilled viscosity), and a defined viscosity increase when the product is consumed and enters the stomach (induced viscosity). Each viscosity characteristic is defined in detail hereinafter.

### A) Room temperature viscosity

The nutritional emulsion embodiments of the present invention have a room temperature or packaged viscosity (at 20° C) that is typically less than about 300 cps, more typically from about 10 cps to about 160 cps, including from about 20 cps to about 70 cps. The room temperature or packaged viscosity may be measured after removing the emulsion from a sealed package such as from a retort processed or aseptically filled can, bottle, or other container.

### B) Chilled Viscosity

The nutritional emulsion embodiments may be chilled prior to use to produce a chilled viscosity as defined herein, which also provides the emulsion with a thick and creamy mouthfeel.

To achieve the chilled viscosity, the nutritional emulsion may be cooled or chilled prior to consumption to a temperature of from about 0° C to about 8° C, including from about 1° C to about 6° C, and also including from about 2° to about 4° C, at which point the viscosity of the emulsion increases by at least about 50 cps, including an increase of from about 100 to about 700 cps, and also including an increase of from about 150 cps to about 350 cps, above the corresponding room temperature viscosity.

The chilled nutritional emulsion, which will therefore have a chilled viscosity that is higher than the room temperature viscosity for the same formula, may therefore have a chilled viscosity of at least about 120 cps, including from about 120 cps to about 600 cps, also including from about 150 cps to about 450 cps, and also including from about 200 cps to about 400 cps.

The chilled viscosity increase is due primarily to the inclusion of the V-complex in the aqueous phase of the nutritional emulsion.

### A) Inducted Viscosity

The induced viscosity provided by these nutritional emulsions helps reduce gastric emptying and blunt the subsequent blood glucose response. The induced viscosity also provides a sense of fullness and enhanced satiety. These induced viscosity nutritional emulsions are especially useful in diabetics and in people interested in maintaining or losing weight.

As induced viscosity beverages, the nutritional emulsions of the present invention have a drinkable viscosity prior to consumption (i.e., packaged viscosity), which then increases in viscosity following consumption and upon entry into the stomach (i.e., induced viscosity). The viscosity increase (induced viscosity) arises primarily from the induced viscosity fiber system (described hereinafter) in the nutritional emulsion.

The nutritional emulsions are further defined by an induced viscosity that is greater than the room temperature viscosity. The induced viscosity is most typically greater than about 300 cps, including at least about 350 cps, including from 400 to 20,000 cps, and also including from about 800 to about 15,000 cps.

Measurement of the induced viscosity depends upon the type of induced viscosity system used. For an emulsion comprising a polymer controlled induced viscosity fiber system, the induced viscosity is measured by adding 20 ③L of bacterial alpha-amylase (Sigma) to 250 grams (g) of the emulsion, shearing the enzyme-treated emulsion for 30 minutes using a Glass-Col mixer, and then measuring viscosity using a Brookfield Viscometer (Model DV-II+) with a 62 spindle at room temperature. This induced viscosity measurement is designed to assimilate the expected induced viscosity for the product following consumption and upon entry into the stomach.

For emulsions comprising an acid-controlled induced viscosity fiber system, the induced viscosity is measured by adding 60 ml of 0.1 N HCL solution to 250 grams of the emulsion, shearing the acidified emulsion for 30 minutes using a Glass-Col mixer, and then measuring the resulting viscosity using a Brookfield (model DVII+) viscometer with a 62 spindle at room temperature.

For other induced viscosity fiber systems, the induced viscosity may be measured by any other in-vitro method suitable for assimilating gastric conditions.

### Induced Viscosity Fiber System

The nutritional emulsion embodiments of the present invention comprise an induced viscosity fiber system, which includes any system that increases the viscosity of the emulsion following consumption, wherein the room temperature or packaged viscosity and the induced viscosity of the emulsions following consumption are within the ranges as defined herein.

Any induced viscosity fiber system that is known or otherwise suitable for safe and effective oral administration are suitable for use herein, some examples of which are described in U.S. Patents 7,183,266 and 7,067,498, and US Patent Publication 20020193344.

Other compositions suitable for use herein include those described in U.S. Patent 6,733,769 (Ryan et al.), US Patent Publications 2005/0233045, 20050170059, 2005/0084592 (Aldred et al.).

### A) Polymer Controlled Induced Viscosity Fiber System

The induced viscosity fiber system may be a polymer controlled induced viscosity fiber system such as that described in U.S. Patent 7,067,498. Such a system comprises a neutral soluble fiber and a partially hydrolyzed starch having a degree of polymerization (DP) of at least 10.

The term "neutral water soluble fiber" as used herein refers to those fibers that can be dissolved in water at room temperature and that carry no charge at a neutral pH.

The nutritional emulsions herein may include those embodiments in which the weight ratio of the neutral soluble fiber to the partially hydrolyzed starch in the polymer controlled induced viscosity fiber system ranges from 0.35:5.0 to 1:5.0, including from 0.7:5.0 to 1:5.0, and also including 1:5.0.

Within a nutritional emulsion containing the polymer controlled induced viscosity fiber system, the neutral soluble fiber is maintained in a dispersed, insoluble state by the presence of the partially hydrolyzed starch. When two or more polymers such as these are present in the same solution, the solubility of the less soluble polymer (i.e., neutral soluble fiber) decreases as the concentration of the more soluble polymer (i.e., partially hydrolyzed starch) increases. However, when the partially hydrolyzed starch is digested by alpha amylase in the stomach, its increasing absence within the stomach allows the neutral soluble fiber within the consumed composition to solubilize and thus form a gel and a higher viscosity composition within the stomach. The resulting viscous mass in the stomach delays gastric emptying and slows or delays glucose absorption

Non limiting examples of neutral soluble fibers for use in the polymer controlled induced viscosity fiber system herein include guar gum, pectin, locust bean gum, methylcellulose, β-glucans, glucomannan, konjac flour, and combinations thereof. Preferred are glucomannan fiber, guar gum, and combinations thereof. The concentration of these neutral soluble fibers are typically at least 0.4%, including from 0.55 to 3.0 %, and also including from 0.65 to 1.5%, by weight of the nutritional emulsion.

Suitable partially hydrolyzed starches for use in this particular induced viscosity fiber system includes those having an average degree of polymerization of at least 10, including at least about 20, also including from about 40 to about 250, and also including from about 60 to about 120, and which are suitable for use in an oral nutritional product. In this context, the degree of polymerization (DP) is the number of glucose or monosaccharide units joined in the molecule. The concentration of the partially hydrolyzed starch is typically at least 2%, including from 3 to 20%, and also including from 3.5 to 6%, by weight of the nutritional emulsion.

Non limiting examples of some suitable partially hydrolyzed starches for use herein include those obtained by acid hydrolysis, enzyme hydrolysis, or both. Most typical for use herein are those having a DP of from 40 to 250, including DP 100 maltodextrin, and other suitable polysaccharides such as inulin, hydrolyzed guar gum, gum Arabic, and combinations thereof.

The partially hydrolyzed starch may also be characterized in terms of dextrose equivalents (DE) rather than DP values, wherein the partially hydrolyzed starch has a DE of less than 10, including from 1 to 8. A dextrose equivalent (DE) is a conventional measurement representing the average reducing power of maltodextrin or other polysaccharide as compared to a dextrose standard. DE values are derived from the formula [DE = 100 + DP], where DP is the degree of polymerization of the maltodextrin or other material, i.e., the number of monosaccharide units in the polysaccharide. For reference, glucose (dextrose) has a DE of 100; starch has a DE of approximately zero.

The induced viscosity fiber system herein includes those embodiments in which the neutral soluble fiber is glucomannan or konjac flour, and the partially hydrolyzed starch is one having a molecular weight of from 1,000 to 50,000 Daltons.

### B) Acid Controlled Induced Viscosity Fiber System

The induced viscosity fiber system for use herein includes acid controlled induced viscosity fiber systems such as those described in U.S. Patent Publication 20020193344. Such a system may comprise a soluble anionic fiber in combination with a water-insoluble, acid-soluble, multivalent cation source.

The term "anionic soluble fiber" as used herein refers to water-soluble fibers that carry negative charges after being dissolved in water at room temperature.

The term "water-insoluble, acid-soluble multivalent cations" refers to salts that are not soluble in water at neutral pH and will react with acid to release the cation. Multivalent cations listed in The Merck Index, Tenth Edition, as insoluble or practically insoluble in water and soluble in acid are examples of suitable salts.

As part of an acid controlled induced viscosity fiber system, the multivalent cations are insoluble in the nutritional emulsion. Following consumption, and upon entering the stomach, the acid-soluble multivalent cations solubilize and dissociate in the acidic environment of the stomach. The dissociated cations then react with and cross link the anionic soluble fiber, which then forms a viscous gel or mass in the stomach. The resulting viscous mass delays gastric emptying and slows or delays glucose absorption.

These acid controlled induced viscosity fiber systems include those embodiments in which the water-insoluble, acid-soluble multivalent cation source represents from 200 to 9000 ppm, including from 300 to 4000 ppm, and also including from 400 to 1000 ppm, by weight of the nutritional emulsion (by weight of the cation).

Non limiting example of suitable water-insoluble, acid-soluble, multivalent cation sources suitable for use in the induced viscosity fiber system include any water-insoluble, acid-soluble salt of magnesium, calcium, iron, chromium, manganese, molybdenum, copper, or zinc, some examples of which include calcium carbonate, calcium fluoride, calcium molybdate, calcium oxalate, calcium phosphate dibasic, calcium phosphate tribasic, calcium pyrophosphate, calcium saccharate, magnesium fluoride, magnesium hydroxide, magnesium oxide, magnesium peroxide, magnesium phosphate tribasic, magnesium pyrophosphate, magnesium selenite, manganese carbonate, manganese oxide, manganese sulfide and combinations thereof. Calcium carbonate and or calcium triphosphate are preferred.

The nutritional emulsions may therefore be substantially free of any water-soluble multivalent cations, or any cations that are otherwise rendered soluble in the finished nutritional product. In this context, the term substantially free means that the nutritional emulsion may contain less than 0.2%, including zero percent, by weight of a water-soluble or product soluble multivalent cation.

These acid controlled induced viscosity fiber systems include those embodiments in which the soluble anionic fiber represents from about 0.2 to about 5%, including form about 0.4 to about 3%, and also including from about 0.8 to about 1.5%, by weight of the nutritional emulsion, non-limiting examples of soluble anionic fibers suitable for use in the induced viscosity fiber system include alginate, low methoxy pectin, carrageenan, xanthan, gellan gum, and combinations thereof. Alginate is particularly useful.

### V-Complex

The nutritional emulsion embodiments of the present invention may be characterized by the presence of a V-complex comprising a food grade surfactant in combination with a polydextrose having an average degree of polymerization of at least about 10. The V-complex may be formed prior to or during the manufacturing process, such as in accordance with the processing methods described herein.

The term "V-complex" as used herein, unless otherwise specified, refers to carbohydrate-surfactant complexes formed by a combination that is substantially free of fat and comprises a food grade surfactant and a polydextrose (i.e., α (1,4) linked glucose polymer) having an average degree of polymerization of at least about 10. In an aqueous liquid, the selected glucose polymers form left-handed, 6-residue helices with a hydrophobic core. Under appropriate processing conditions, this hydrophobic core traps the hydrophobic section of the food grade surfactant to form a carbohydrate-surfactant complex having a distinctive V-complex x-ray diffraction pattern. This type of complex is referred to herein as a V-complex.

The nutritional emulsions may be evaluated for the presence of the V-complex. This may be done indirectly by either measuring the viscosity change when the product is refrigerated or cooled as described herein, and or by evaluating the product by conventional x-ray diffraction methods for the presence of the V-complex. Such x-ray diffraction methods are described, for example, by J-L Jane and Robyt, J. (1984) Carbohydrate Research 132:105. Journal of Rheology -- May 1998 -- Volume 42, Issue 3, pp. 507-525 Mercier, C., R. Charbonniere, J. Grebaut, and J. F. de La Guerivière.

Inclusion or formation of the V-complex in the nutritional emulsion affects the resulting rheology profile of the emulsion. The nutritional emulsion, with the V-complex in the aqueous phase of the emulsion, has a relatively low viscosity at room temperature (20° C), but when chilled has a significantly higher viscosity as the temperature drop further facilitates the formation of the V-complex within the aqueous phase, which then imparts both viscosity and a creamy mouthfeel to the emulsion. The lower viscosity during manufacture allows for reduced processing or sterilization temperatures. This may reduce manufacturing costs as well as reduce the rate or extent of formation of undesirable Malliard reaction products in the finished product.

The V-complex in the nutritional emulsions provides a creamy mouthfeel when consumed. In this context, the term "creamy" means that the product has a mouthfeel similar to that of a similar nutritional emulsion having a higher fat content. The nutritional emulsions are therefore especially useful when formulated as a low fat formulation since the V-complex compensates for the watery-mouthfeel commonly associated with reduced fat content in an emulsion.

The V-complex may be formed within the nutritional emulsions by methods described herein. This typically involves combining the food grade surfactant with the polydextrose in a separate aqueous slurry, substantially free of fat, that is then used to form part or all of the aqueous phase of the emulsion. Thus, the selected food grade surfactant and polydextrose may be dispersed in an aqueous slurry during processing, under conditions that melt and disperse the food grade surfactant throughout the aqueous slurry, and thereafter combined and homogenized with other fat and protein ingredients to form a nutritional emulsion.

It should be noted, however, that the nutritional emulsion may contain other surfactants in addition to the food grade surfactant in the aqueous phase, especially to help emulsify the oil component in the emulsion, but these oil phase surfactants do not form the desired V-complex with the polydextrose as described herein. Oil blends added to the nutritional emulsion typically contain from 1 to 6% surfactants by weight of the oil.

The term "substantially free of fat" as used herein means that the referenced material, either the aqueous phase of the nutritional emulsion or the aqueous slurry used in preparation of the nutritional emulsion, contains less than about 0.1%, including less than 0.05%, and also including zero percent by weight of fat. It is understood, however, that such exclusion does not apply to the food grade surfactant, which in the case of an acylglycerol could be considered a fat.

The aqueous slurry comprising the food grade surfactant and the selected carbohydrate are typically heated to melt the surfactants and mixed sufficiently to disperse or dissolve the surfactant and selected carbohydrate, to thus promote the interaction of those ingredients to form the desired V-complexes therefrom. The resulting v-complex slurry may then be added with other ingredients in accordance with conventional or otherwise known processing steps for manufacturing the desired nutritional emulsion. The aqueous slurry is most typically heated to a temperature above the melt point of the surfactant, which heat may be added in the form of a heated carbohydrate mixture added to the surfactant, with a subsequent melting of the surfactant in newly formed aqueous slurry.

When the nutritional emulsion is later cooled or chilled prior to consumption, conditions favor further formation of the V-complex, which results in a surprising increase in product viscosity and creamy mouthfeel. Because the resulting V-complexes are essentially tiny particles that can be digested by saliva enzymes, they impart a thick, creamy mouthfeel similar to that of a rich oil-in-water type emulsion, e.g., milk-based or other fat based emulsion, even when the nutritional emulsion contains relatively low fat levels (which should otherwise result in a thin, watery mouthfeel).

The nutritional emulsion embodiments of the present invention, however, are distinct from the many prior art compositions that merely comprise food grade surfactants and a polydextrose, but do not combine the latter two ingredients in the aqueous phase of an emulsion or otherwise form a V-complex in that aqueous phase. In other words, the mere inclusion of these two ingredients in a composition is not sufficient to achieve the desired viscosity benefit, unless they are also combined or otherwise complexed within the aqueous phase of the nutritional emulsion.

The polydextrose component of the aqueous phase or of the V-complex has an average degree of polymerization of at least about 10, including from about 20 to about 400, also including from about 40 to about 200, and also including from about 60 to about 100. For purposes of defining the inventions hereof, the terms "degree of polymerization" and "average degree of polymerization" are used interchangeably to mean an average degree of polymerization value. The degree of polymerization (DP) is an art recognized term referring to the number of glucose or monomer units in a polymer.

Suitable polydextrose for use herein may include any glucose polymer having the requisite degree of polymerization that is also safe for use in oral nutritional products. Especially useful are maltodextrins and starches.

Suitable maltodextrins for use herein are those that are safe for use in oral nutritional products and that also have the requisite DP value, non limiting examples of which include Maltrin® M040 (DE range 4-7), Maltrin® M050 (DE range 4-7), Maltrin® 070 (DE range 6-9), Maltrin® M440 (DE range 4-7), all available from Grain Processing Corporation, Muscatine, Iowa, USA. In this context, DE refers to the dextrose equivalent of the maltodextrin. DE values correlate with DP values in accordance with the equation DP = 100 / DE.

Starch suitable for use in forming the V-complex may include regular starches, modified starches such as cold water soluble starches, pregelatinized starches or acid thinned starches.

Food grade surfactants for use herein include those surfactants that are suitable for use in an oral nutritional and that comprise at least one hydrophobic moiety, typically a hydrocarbon carbon. Non limiting examples of such surfactants include mono- and diacylglycerol esters of one or more fatty acids having 12 or more carbon atoms, including from 12 to 24 carbon atoms, and also including from 18 to 22 carbon atoms, specific non-limiting examples of which include lauric acid, myristic acid, palmitic acid, stearic acid, oleic acid, linoleic acid, linolenic acid, arachidonic acid, eicosapentaenoic acid, docosahexaenoic acid, and behenic acid. These acylglycerols and methods of preparing them are well known in the formulation arts, all of which may be used herein in preparation of the food grade surfactants for use in the nutritional emulsion embodiments and methods of the present invention.

Specific non-limiting examples of suitable acylglycerols include Myverol^{™} 18-06 monoacylglycerol (distilled monoglycerides from hydrogenated soybean oil - Foodpro Co., Dubai, United Arab Emirates), Dimodan S K-A and Dimodan R/D K-A (Danisco), and BFP 65 PLM (American Ingredients). Specific non-limiting examples of other suitable food grade surfactants for use herein include sodium stearoyl-2 lactylate (SSL), sucrose esters, diacetyl tartaric acid esters, and combinations thereof.

Other non-limiting examples of suitable food grade surfactants are described in US Patent 5,645,856, which descriptions are incorporated herein by reference. Non limiting examples of such surfactants include glyceryl mono-/di-caprylate, glyceryl mono-di-caprylate/caprate, glyceryl mono-caprylate, glyceryl mono-stearate, glyceryl mono-/di-ricinoleate, glyceryl caprylate/caprate, glyceryl mono-oleate), glyceryl dilaurate, glyceryl mono-oleate, distilled monoglycerides from sunflower oil, and combinations thereof.

Other suitable food grade surfactants include acetic, succinic, lactic, citric and/or tartaric esters of mono- and/or di-glycerides of fatty acids, e.g., distilled acetylated monoglycerides, caprylic/capric diglyceryl succinate, mono/di-succinylated monoglycerides, glyceryl stearate citrate, glyceryl monostearate/citrate/lactate, diacetyl tartaric esters of monoglycerides, and combinations thereof.

The amount of acylglycerols or other food grade surfactants for use in the nutritional emulsions should be sufficient to form the V-complex within the aqueous phase of the emulsions. Such amounts may comprise at least about 0.003%, including from about 0.1 to about 5%, also including from about 0.2 to about 1%, by weight of the nutritional emulsion. It should be noted, however, that the nutritional emulsion may further comprise additional food grade surfactants for purposes other than forming the V-complex, for example as emulsifying agents for the nutritional emulsion or components thereof.

The amount of the selected polydextrose for use in the nutritional emulsions should be sufficient to form the V-complex within the aqueous phases of the emulsions. Such amounts may comprise at least about 0.5%, including from about 0.75 to about 20%, also including from about 1 to about 5%, and also including from about 1.5 to about 3.5%, by weight of the nutritional emulsion. It should be noted, however, that the nutritional emulsion may further comprise additional starch, maltodextrins, or other carbohydrates, including those having average DP values below about 10 as well as those having DP values above about 10, including those having DP values from about 10 to about 400.

The resulting weight ratio of the selected polydextrose to the food grade surfactant component of the formed complex may vary depending upon the selected formulation, including the selected carbohydrates and surfactants in the V-complex. Such ratios most typically range up to about 50:1, including from about 20:1 to about 5:1, and also including from about 10:1 to about 6:1.

### Macronutrients

The nutritional emulsion embodiments of the present invention comprise fat, protein, and carbohydrate macronutrients, all in addition to or inclusive of polydextrose and food grade surfactant components of the aqueous phase or V-complex, and the induced viscosity fiber system, all as described herein. Any source of such nutrients that is known or otherwise suitable for use in an oral nutritional product is also suitable for use herein, provided that such nutrients are also compatible with the other selected ingredients in the formulation.

Although concentrations or amounts of each macronutrient may vary depending upon the nutritional needs of the intended user, such concentrations or amounts most typically fall within one of the following embodied ranges (inclusive of the components of the polydextrose and food grade surfactant or V-complex).

| Macronutrient | Embodiments | | |
|---|---|---|---|
| | A | B | C |
| Carbohydrate¹ - % total calories | 10-85 | 20-60 | 40-60 |
| Fat² - % total calories | 10-85 | 10-50 | 15-35 |
| Protein - % total calories | 5-80 | 10-30 | 15-25 |
| | | | |
| Carbohydrate¹ g/100ml | 1-40 | 4-30 | 10-20 |
| Fat² g/100ml | 0.2-30 | 0.5-15 | 1-5 |
| Protein g/100ml | 0.5-30 | 1-15 | 2-10 |

| | | | |
|---|---|---|---|
| 1. Includes polydextrose component of the aqueous phase or V-complex 2. Includes food grade surfactants of the aqueous phase or V-complex | | | |

Non-limiting examples of suitable fat sources for use herein may include coconut oil, fractionated coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, marine oils, cottonseed oils, and combinations.

Non-limiting examples of suitable carbohydrate sources for use herein may include hydrolyzed or modified starch or cornstarch, glucose polymers, corn syrup, corn syrup solids, rice-derived carbohydrate, glucose, fructose, lactose, high fructose corn syrup, indigestible oligosaccharides (e.g., fructooligosaccharides), honey, sugar alcohols (e.g., maltitol, erythritol, sorbitol), and combinations thereof.

Suitable protein sources for use herein include hydrolyzed, partially hydrolyzed or non-hydrolyzed proteins or protein sources, which may be derived from any known or otherwise suitable source such as milk (e.g., casein, whey), animal (e.g., meat, fish), cereal (e.g., rice, corn), vegetable (e.g., soy), or combinations thereof, non-limiting examples of such proteins include milk protein isolates, casein protein isolates, milk protein concentrate, whole cows milk, partially or completely defatted milk, soy protein isolates, and so forth.

The nutritional emulsions may also be formulated as low fat emulsions comprising from about 0.1 to about 2.0 grams, including from about 0.5 to about 1.5 grams, and also including from about 0.75 to about 1.1 grams, of fat per 100 ml of the emulsion, and /or from about 1% to about 20%, including from about 3% to about 10%, and also including from about 4% to about 8%, fat as a percentage of total calories.

The nutritional emulsions may also be formulated as a low calorie product that comprises from about 50 to about 200 kcals, including from about 75 to about 170, and also including from about 99 to about 140 kcal, per 240 ml of the emulsion.

### Optional Ingredients

The nutritional emulsion embodiments of the present invention may further comprise other optional components that may modify the physical, chemical, aesthetic or processing characteristics of the products or serve as pharmaceutical or additional nutritional components when used in the targeted population. Many such optional ingredients are known or otherwise suitable for use in other nutritional products and may also be used in the compositions herein, provided that such optional ingredients are safe and effective for oral administration and are compatible with the essential and other ingredients in the selected product form.

Non-limiting examples of such optional ingredients include preservatives, antioxidants, other additional emulsifying agents, buffers, pharmaceutical actives, additional nutrients as described herein, sweeteners including artificial sweeteners (e.g., saccharine, aspartame, acesulfame K, sucralose) colorants, flavors, thickening agents and stabilizers, and so forth.

The nutritional emulsion embodiments of the present invention may further comprise any of a variety of other vitamins or related nutrients, non-limiting examples of which include vitamin A, vitamin D, vitamin E, vitamin K, thiamine, riboflavin, pyridoxine, vitamin B12, carotenoids, niacin, folic acid, pantothenic acid, biotin, vitamin C, choline, inositol, salts and derivatives thereof, and combinations thereof.

The nutritional emulsion embodiments may further comprise any of a variety of other additional minerals, non-limiting examples of which include calcium, phosphorus, magnesium, iron, zinc, manganese, copper, sodium, potassium, molybdenum, chromium, selenium, chloride, and combinations thereof.

### MANUFACTURE

The nutritional emulsions may be manufactured by any conventional or otherwise known method for making nutritional emulsions, most typically for making nutritional emulsions or milk based emulsions, except that the formulation should include or accommodate the process steps described herein for the formation of the specified aqueous phase or V-complex.

Most typically, two or more separate slurries are prepared, one of which must be an aqueous carbohydrate slurry (aqueous slurry) that is substantially free of fat and comprises the food grade surfactant in combination with the selected maltodextrin or starch. Other slurries may include a protein-in-oil slurry (e.g., protein, oil, emulsifier or surfactant in addition to the food grade surfactant in the aqueous slurry), a protein in water slurry (e.g., protein, water), and additional carbohydrate slurries. The multiple slurries are eventually combined together in a blend tank, subjected to ultra high temperature processing, homogenized, infused with added vitamins or other optional ingredients, diluted with water as appropriate. The resulting nutritional emulsions may then be aseptically packaged or otherwise filled into retort stable packages and then subjected to retort sterilization.

The nutritional emulsion embodiments of the present invention may be prepared so that the food grade surfactant and polyglucose, or the V-complex, are formulated into the aqueous phase of the emulsion. This may be accomplished, specifically, by inclusion of the following steps in the selected manufacturing process:
(a) forming an aqueous slurry, substantially free of fat, by combining a food grade surfactant with a polydextrose having an average degree of polymerization of at least about 10;
(b) combining and homogenizing the aqueous slurry with fat and protein to form a nutritional emulsion having an aqueous phase comprising from about 10% to 100% by weight of the food grade surfactant and from about 10% to 100% by weight of the polydextrose, wherein the nutritional emulsion has a first viscosity of less than about 300 cps as measured at 20° C and a second viscosity as measured at between about 0° C and about 8° C that is at least 50 cps higher than the first viscosity.

The nutritional emulsion embodiments may be also prepared by inclusion of the following steps in the selected manufacturing process:
(a) forming an aqueous slurry, substantially free of fat, by combining a food grade surfactant with a polydextrose having an average degree of polymerization of at least about 10;
(b) combining and homogenizing the aqueous slurry with fat and protein to form a nutritional emulsion having an aqueous phase comprising a V-complex containing at least some of the food grade surfactant complexed with the polydextrose, most typically from about 10% to 100% by weight of each, wherein the nutritional emulsion has a first viscosity of less than about 300 cps as measured at 20° C and a second viscosity as measured at between about 0° C and about 8° C that is at least 50 cps higher than the first viscosity.

The induced viscosity fiber system may be incorporated, in whole or in part, in the aqueous slurry within which the V-complex is formed. The polyglucose for use in forming the V-complex may form some or all of the lightly hydrolyzed starch (e.g., maltodextrin with selected DP) of the induced viscosity system.

The above method embodiments may be modified to also include the various elements or features of the nutritional emulsion embodiments as described herein.

The manufacturing methods typically further comprise providing physical shear or mixing, while also heating or supplying heat, to the aqueous slurry sufficient to melt the food grade surfactant and solubilize the polydextrose, and thus disperse the two ingredients throughout the aqueous slurry.

The method embodiments may further comprise packaging the resulting nutritional emulsion in a suitable container. The method may also further comprise exposing the packaged nutritional emulsion to retort sterilization to produce a retort packaged nutritional emulsion having a first viscosity (packaged or room temperature viscosity) and second viscosity (chilled viscosity) as defined herein. Retort sterilization is a process step well known to one of ordinary skill in the formulation art, which typically involves high temperature treatment of a packaged liquid nutritional. The nutritional emulsion may also be aseptically packaged rather than retort sterilized.

The method embodiments of the present invention may further comprise the following steps, or instructions to a user or consumer to perform the following steps, wherein such steps may include 1) cooling or refrigerating the nutritional emulsion, or packaged nutritional emulsion, prior to use, or 2) cooling or refrigerating the nutritional emulsion, or the retort packaged nutritional emulsion, to a temperature sufficient to increase the viscosity of the emulsion by at least about 50 cps, which may include an increase of from about 100 to about 700 cps, and may also include an increase of from about 150 cps to about 350 cps, above the first viscosity as measured at 20° C. To achieve the desired viscosity increase, the nutritional emulsion is most typically cooled to between about 0° C to about 8° C, which may include a temperature of from about 1° C to about 6° C, and may also include a temperature of from about 2° to about 4° C.

The chilled nutritional emulsion, which therefore has a surprisingly higher viscosity than the nutritional emulsion at room temperature, typically has a chilled viscosity of at least about 120 cps, including from about 120 cps to about 600 cps, including from about 150 cps to about 450 cps, and also including from about 200 cps to about 400 cps.

When the nutritional emulsion is then cooled or chilled prior to consumption, conditions favor further formation or development of the V-complex, which results in a surprising increase in product viscosity and creamy texture. Because the resulting V-complexes are essentially tiny particles that can be digested by saliva enzymes, they impart a thick, creamy mouthfeel, even when there may be a low fat level in the nutritional emulsion.

### EXAMPLES 1-3

The following examples illustrate specific embodiments of the nutritional emulsions of the present invention, including suitable techniques to prepare the emulsions. Each represents a 1000 kg batch. The examples are given solely for the purpose of illustration and are not to be construed as limitations of the present invention.

| **Ingredient** | **Example 1 (kg)** | **Example 2 (kg)** | **Example 3 (kg)** |
|---|---|---|---|
| **Protein-in-water slurry** | | | |
| Water | 475.30 | 475.30 | 475.30 |
| MPI | 21.69 | 21.69 | 21.69 |
| Caseinate | 5.68 | 5.68 | 5.68 |
| Hydrolyzed Na Caseinate | 9.53 | 9.53 | 9.53 |
| **Aqueous slurry (carbohydrate slurry)** | | | |
| Water | 186.20 | 186.20 | 186.20 |
| Potassium Citrate | 0.550 | 0.550 | 0.550 |
| Na Citrate | 2.40 | 2.40 | 2.40 |
| Gellan gum | 0.200 | 0.200 | 0.200 |
| Avicel | 0.530 | 0.530 | 0.530 |
| UTM/TM premix | 0.270 | 0.270 | 0.270 |
| K Iodide | 0.00016 | 0.00016 | 0.00016 |
| Cr Chloride | 0.00016 | 0.00016 | 0.00016 |
| Mg Chloride | 0.400 | 0.400 | 0.400 |
| K Chloride | 1.20 | 1.20 | 1.20 |
| m-TCP | 0.900 | 0.900 | 0.900 |
| Mg Phosphate Dibasic | 3.10 | 3.10 | 3.10 |
| K Phosphate Dibasic | 0.350 | 0.350 | 0.350 |
| Calcium Carbonate | 0.800 | 0.800 | 0.800 |
| DE-1 Maltodextrin | 50.53 | 52.63 | 52.63 |
| M-200 Maltodextrin | 19.32 | 0.00 | 0.00 |
| DE-3 Maltodextrin | 0.00 | 11.58 | 11.58 |
| Fructose | 28.00 | 28.00 | 28.00 |
| Glycerol | 16.00 | 16.00 | 16.00 |
| Monoacylglycerol | 1.50 | 7.5 | 0.00 |
| Guar gum | 3.33 | 10.0 | 10.0 |
| Konjac Flour | 5.56 | 0.00 | 0.00 |
| **Oil blend or slurry** | | | |
| Enova Oil | 7.42 | 7.42 | 7.42 |
| Canola Oil | 5.19 | 5.19 | 5.19 |
| HOSO | 10.88 | 10.88 | 10.88 |
| Soy Lecithin | 1.24 | 1.24 | 1.24 |
| Vitamin A Palmitate | 0.0077 | 0.0077 | 0.0077 |
| Vitamin E | 0.07194 | 0.07194 | 0.07194 |
| Vitamin DEK premix | 0.04747 | 0.04747 | 0.04747 |
| Lutein | 0.05035 | 0.05035 | 0.05035 |
| Cocoa Powder | 10.53 | 10.53 | 10.53 |
| Monoacylglycerol | 0.00 | 0.00 | 7.8 |
| **Vitamins and water dilution** | | | |
| Water | 7.55 | 7.55 | 7.55 |
| Ascorbic Acid | 0.4248 | 0.4248 | 0.4248 |
| Vitamin Premix | 0.07086 | 0.07086 | 0.07086 |
| Choline Chloride | 0.3433 | 0.3433 | 0.3433 |
| Sucralose | 0.37000 | 0.37000 | 0.37000 |
| Natural and Artificial Flavors | 1.2 | 1.2 | 1.2 |
| Dilution Water | 71.6 | 71.6 | 71.6 |

In preparing this nutritional emulsion (-1000 kg), the fat blend is formed separately by combining the specified ingredients. The protein-in-water slurry is also prepared separately by combining the specified ingredients. The aqueous slurry (carbohydrate slurry) is likewise formed as a separate mixture by combining the specified ingredients with sufficient heat and shear to melt the food grade surfactant and dissolve/disperse the carbohydrate solids.

The carbohydrate slurry is then added to the protein-in-water slurry and the blend pH adjusted to 6.7-7.0. To the resulting blend is added the fat blend. The resulting fat-containing mixture is then processed at UHT temperatures (295°F for 5 seconds) and homogenized at 4000 psi. The ingredients for the vitamin solution are then combined and the pH adjusted to 6.5-7.5 using 45% KOH. The resulting vitamin blend is then added to the homogenized blend at standardization. The final blend is then packaged and sealed in individual 8 oz. containers and subjected to retort.

The packaged nutritional emulsion is characterized by the presence of an aqueous phase having a V-complex comprising the food grade surfactant complexed with the selected maltodextrin component, or by the presence within the aqueous phase of at least about 10% by weight of the food grade surfactant and at least about 10% by weight of the polydextrose (maltodextrin). The retort-packaged products are then labeled with instruction to cool or refrigerate prior to use. The nutritional emulsions are removed from the package and tested for the presence of the v-complex.

Each of the resulting retort packaged nutritional emulsions (Examples 1-3) has a viscosity as measured at 20° C of between about 20 and 160 cps. Each is refrigerated to between 0° and 8° C and develops a chilled viscosity of between about 220 and about 350 cps that is then consumed as chilled. The chilled emulsion has a thick, creamy mouthfeel.

Each of the resulting retort packaged nutritional emulsions has an induced viscosity in accordance with the viscosity methods herein, such that the induced viscosity is between about 600 and 10,000 cps. Once consumed, the nutritional emulsions provide a feeling of fullness for between about 4 and about 8 hours, and also provide a blunted glycemic response. The nutritional emulsions are used to control blood glucose levels in a diabetic. The nutritional emulsions are also used to help manage appetite and weight gain

## Claims

1. Nutritional emulsions comprising fat, protein, and carbohydrate, including:
(A) an induced viscosity fiber system, and
(B) a carbohydrate-surfactant complex (V-complex) located within an aqueous phase of the emulsion and comprising a food grade surfactant complexed with a polydextrose having an average degree of polymerization of at least about 10,
wherein the nutritional emulsions have a first viscosity at 20° C of less than about 100 cps, a second viscosity at a temperature of from 0° C to 8° C that is at least about 50 cps higher than said first viscosity, and an induced viscosity of at least about 300 cps.

2. The nutritional emulsion of claim 1 wherein the emulsion comprises, as a percentage of total calories, from 10 to 85% carbohydrate, from 10 to 85% fat, and from 5 to 40% protein.

3. The nutritional emulsion of claim 1 wherein the induced viscosity fiber system comprises, by weight of the emulsion, from 0.2 to 5% of a soluble anionic fiber source and from 200 to 9000 ppm of a water-insoluble, acid-soluble, multivalent cation.

4. The nutritional emulsion of claim 3 wherein the emulsion is substantially free of soluble multivalent cations.

5. The nutritional emulsion of claim 3 wherein the soluble anionic fiber is selected from the group consisting of alginate, low methoxy pectin, carrageenan, xanthan, gellan gum, and combinations thereof.

6. The nutritional emulsion of claim 1 wherein the induced viscosity fiber system comprises, by weight of the emulsion, at least 0.4% of a neutral soluble fiber and at least 2% of a partially hydrolyzed starch having a degree of polymerization of at least 10.

7. The nutritional emulsion of claim 6 wherein the neutral soluble fiber is selected from the group consisting of guar gum, high-methoxy pectin, locust bean gum, methylcellulose, beta-glucans, glucomannan, konjac flour, and combinations thereof.

8. The nutritional emulsion of claim 7 wherein the partially hydrolyzed starch has a degree of polymerization of from about 40 to about 250.

9. The nutritional emulsion of claim 1 wherein the emulsion has an induced viscosity of from 400 to 20,000 cps.

10. A nutritional emulsion according to claim 1 wherein the food grade surfactant is a monoacylglyerol.

11. A nutritional emulsion according to claim 1 wherein polydextrose comprises maltodextrin.

12. A nutritional emulsion according to claim 1, wherein the V-complex comprises a C12 or higher monoacylglycerol in combination with maltodextrin having an average degree of polymerization of from about 20 to about 400.

13. A nutritional emulsion according to claim 1 wherein the nutritional emulsion comprises from about 1% to about 5% by weight of the polydextrose, and from about 0.001 % to about 5% by weight of the food grade surfactant.

14. A nutritional emulsion according to claim 1 wherein the nutritional emulsion has a weight ratio of the polydextrose to the food grade surfactant of from about 50 :1 to about 2 :1.

15. A nutritional emulsion according to claim 1 wherein the nutritional emulsion has a first viscosity at 20°C of less than about 70 cps and a second viscosity at a temperature of from 0°C to 8°C that is from about 100 to about 350 cps higher than said first viscosity.

## Patentansprüche

1. Ernährungsemulsionen umfassend Fett, Protein, und Kohlenhydrat, einschließlich:
(A) einem induzierte-Viskosität-Ballaststoffsystem, und
(B) einem Kohlenhydrat-Tensid-Komplex (V-Komplex), angeordnet innerhalb einer wässerigen Phase der Emulsion und umfassend ein Tensid in Lebensmittelqualität, komplexiert mit einer Polydextrose, die einen durchschnittlichen Polymerisationsgrad von mindestens ungefähr 10 hat,
worin die Ernährungsemulsionen eine erste Viskosität bei 20°C von weniger als ungefähr 100 cps haben, eine zweite Viskosität bei einer Temperatur von 0°C bis 8°C, die mindestens ungefähr 50 cps höher ist als die erste Viskosität, und eine induzierte Viskosität von mindestens ungefähr 300 cps.

2. Die Ernährungsemulsion gemäß Anspruch 1, worin die Emulsion, als einen Prozentsatz der Gesamtkalorien, von 10 bis 85% Kohlenhydrat, von 10 bis 85% Fett, und von 5 bis 40% Protein umfasst.

3. Die Ernährungsemulsion gemäß Anspruch 1, worin das induzierte-Viskosität-Ballaststoffsystem, bezogen auf das Gewicht der Emulsion, von 0,2 bis 5% einer löslichen anionischen Ballaststoffquelle und von 200 bis 9000 ppm eines wasserunlöslichen, säurelöslichen, multivalenten Kations umfasst.

4. Die Ernährungsemulsion gemäß Anspruch 3, worin die Emulsion im Wesentlichen frei von löslichen multivalenten Kationen ist.

5. Die Ernährungsemulsion gemäß Anspruch 3, worin der lösliche anionische Ballaststoff gewählt ist aus der Gruppe bestehend aus Alginat, Nieder-Methoxy-Pectin, Carrageen, Xanthan, Gellangummi, und Kombinationen daraus.

6. Die Ernährungsemulsion gemäß Anspruch 1, worin das induzierte-Viskosität-Ballaststoffsystem, bezogen auf das Gewicht der Emulsion, mindestens 0,4% eines neutralen löslichen Ballaststoffs und mindestens 2% einer teilweise hydrolysierten Stärke mit einem Polymerisationsgrad von mindestens 10 umfasst.

7. Die Ernährungsemulsion gemäß Anspruch 6, worin der neutrale lösliche Ballaststoff gewählt ist aus der Gruppe bestehend aus Guargummi, Hoch-Methoxy-Pectin, Johannisbrotkernmehl, Methylzellulose, Beta-Glucanen, Glucomannan, Teufelszungen(konjac)mehl, und Kombinationen daraus.

8. Die Ernährungsemulsion gemäß Anspruch 7, worin die teilweise hydrolysierte Stärke einen Polymerisationsgrad von ungefähr 40 bis ungefähr 250 hat.

9. Die Ernährungsemulsion gemäß Anspruch 1, worin die Emulsion eine induzierte Viskosität von 400 bis 20,000 cps hat.

10. Eine Ernährungsemulsion gemäß Anspruch 1, worin das Tensid in Lebensmittelqualität ein Monoacylglycerol ist.

11. Eine Ernährungsemulsion gemäß Anspruch 1, worin Polydextrose Maltodextrin umfasst.

12. Eine Ernährungsemulsion gemäß Anspruch 1, worin der V-Komplex ein C12 oder höheres Monoacylglycerol in Kombination mit Maltodextrin mit einem durchschnittlichen Polymerisationsgrad von ungefähr 20 bis ungefähr 400 umfasst.

13. Eine Ernährungsemulsion gemäß Anspruch 1, worin die Ernährungsemulsion von ungefähr 1% bis ungefähr 5% bezogen auf das Gewicht der Polydextrose, und von ungefähr 0,001% bis ungefähr 5% bezogen auf das Gewicht des Tensids in Lebensmittelqualität umfasst.

14. Eine Ernährungsemulsion gemäß Anspruch 1, worin die Ernährungsemulsion ein Gewichtsverhältnis der Polydextrose zu dem Tensid in Lebensmittelqualität von ungefähr 50:1 bis ungefähr 2:1 hat.

15. Eine Ernährungsemulsion gemäß Anspruch 1, worin die Ernährungsemulsion eine erste Viskosität bei 20°C von weniger als ungefähr 70 cps hat, und eine zweite Viskosität bei einer Temperatur von 0°C bis 8°C, die von ungefähr 100 bis ungefähr 350 cps höher ist als die erste Viskosität.

## Revendications

1. Émulsions nutritionnelles comprenant une graisse, une protéine, et un glucide, comprenant :
(A) un système de fibre à viscosité induite, et
(B) un complexe glucide-tensioactif (complexe V) localisé dans une phase aqueuse de l'émulsion et comprenant un tensioactif de qualité alimentaire en complexe avec un polydextrose ayant de degré de polymérisation moyen d'au moins environ 10,
où les émulsions nutritionnelles possèdent une première viscosité à 20 °C qui est inférieure à environ 100 cps, une seconde viscosité à une température comprise entre 0 °C et 8 °C qui est au moins environ 50 cps plus élevée que ladite première viscosité, et une viscosité induite d'au moins environ 300 cps.

2. Émulsion nutritionnelle selon la revendication 1, où l'émulsion comprend, en tant que pourcentage des calories totales, entre 10 et 85 % de glucide, entre 10 et 85 % de graisse, et entre 5 et 40 % de protéine.

3. Émulsion nutritionnelle selon la revendication 1, dans laquelle le système de fibre à viscosité induite comprend, en poids de l'émulsion, entre 0,2 et 5 % d'une source de fibre anionique soluble et entre 200 et 9000 ppm d'un cation multivalent insoluble dans l'eau et soluble dans un acide.

4. Émulsion nutritionnelle selon la revendication 3, où l'émulsion est essentiellement exempte de cations multivalents solubles.

5. Émulsion nutritionnelle selon la revendication 3, dans laquelle la fibre anionique soluble est sélectionnée dans le groupe consistant en l'alginate, la pectine faiblement méthoxylée, le carraghénane, le xanthane, la gomme gellane, et des combinaisons de ceux-ci.

6. Émulsion nutritionnelle selon la revendication 1, dans laquelle le système de fibre à viscosité induite comprend, en poids de l'émulsion, au moins 0,4 % d'une fibre soluble neutre et au moins 2 % d'un amidon partiellement hydrolysé ayant un degré de polymérisation d'au moins 10.

7. Émulsion nutritionnelle selon la revendication 6, dans laquelle la fibre soluble neutre est sélectionnée dans le groupe consistant en la gomme de guar, la pectine hautement méthoxylée, la gomme de caroube, la méthylcellulose, des bêta-glucanes, le glucomannane, la farine de konjac, et des combinaisons de ceux-ci.

8. Émulsion nutritionnelle selon la revendication 7, dans laquelle l'amidon partiellement hydrolysé possède un degré de polymérisation d'environ 40 à environ 250.

9. Émulsion nutritionnelle selon la revendication 1, où l'émulsion possède une viscosité induite de 400 à 20 000 cps.

10. Émulsion nutritionnelle selon la revendication 1, dans laquelle le tensioactif de qualité alimentaire est un monoacylglycérol.

11. Émulsion nutritionnelle selon la revendication 1, dans laquelle le polydextrose comprend la maltodextrine.

12. Émulsion nutritionnelle selon la revendication 1, dans laquelle le complexe V comprend un monoacylglycérol en C12 ou plus en combinaison avec une maltodextrine ayant un degré de polymérisation moyen d'environ 20 à environ 400.

13. Émulsion nutritionnelle selon la revendication 1, où l'émulsion nutritionnelle comprend entre environ 1 % et environ 5 % en poids du polydextrose, et entre environ 0,001 % et environ 5 % en poids du tensioactif de qualité alimentaire.

14. Émulsion nutritionnelle selon la revendication 1, où l'émulsion nutritionnelle possède un rapport en poids du polydextrose sur le tensioactif de qualité alimentaire d'environ 50:1 à environ 2:1.

15. Émulsion nutritionnelle selon la revendication 1, où l'émulsion nutritionnelle possède une première viscosité à 20 °C qui est inférieure à environ 70 cps et une seconde viscosité à une température comprise entre 0°C et 8 °C qui est entre environ 100 et environ 350 cps plus élevée que ladite première viscosité.
